(19) Europäisches Patentamt / European Patent Office / Office européen des brevets

(11) EP 2 559 769 A1

(12) **EUROPEAN PATENT APPLICATION**
published in accordance with Art. 153(4) EPC

(43) Date of publication:
**20.02.2013 Bulletin 2013/08**

(21) Application number: **11768814.3**

(22) Date of filing: **11.04.2011**

(51) Int Cl.:
***C12P 13/00*** *(2006.01)* ***C12N 15/09*** *(2006.01)*
***C07C 211/09*** *(2006.01)* ***C12N 9/88*** *(2006.01)*

(86) International application number:
**PCT/JP2011/058987**

(87) International publication number:
**WO 2011/129293 (20.10.2011 Gazette 2011/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.04.2010 JP 2010091602**

(71) Applicant: **Toray Industries, Inc.**
**Tokyo 103-8666 (JP)**

(72) Inventors:
- **SAWAI, Kenji**
  **Kamakura-shi, Kanagawa 248-8555 (JP)**
- **WATANABE, Shiomi**
  **Kamakura-shi, Kanagawa 248-8555 (JP)**
- **MIMITSUKA, Takashi**
  **Kamakura-shi, Kanagawa 248-8555 (JP)**
- **SAWAI, Hideki**
  **Kamakura-shi, Kanagawa 248-8555 (JP)**

(74) Representative: **Kador, Utz Ulrich**
**Kador & Partner**
**Corneliusstrasse 15**
**80469 München (DE)**

# (54) METHOD FOR PRODUCING 1,5-PENTANEDIAMINE

(57) A method for producing 1,5-pentanediamine by fermentation using a microorganism which has an LDC gene in the chromosome and whose by-production of L-lysine is suppressed is disclosed. The method for producing 1,5-pentanediamine is a method for producing 1,5-pentanediamine by coryneform bacterium having a gene encoding lysine decarboxylase in its chromosome, which coryneform bacterium maintains a lysine decarboxylase activity of not less than 50 mU/mg protein during culturing.

EP 2 559 769 A1

SEQUENCE LISTING

<110> TORAY INDUSTRIES, INC.

<120> Process of Producing 1,5-pentandiamine

<130> PF000430-PCT

<160> 28

<170> PatentIn version 3.1

<210> 1
<211> 2148
<212> DNA
<213> Escherichia coli

<400> 1
```
atgaacgtta ttgcaatatt gaatcacatg gggtttatt ttaaagaaga acccatccgt      60

gaacttcatc gcgcgcttga acgtctgaac ttccagattg tttacccgaa cgaccgtgac    120

gacttattaa aactgatcga aaacaatgcg cgtctgtgcg gcgttatttt tgactgggat    180

aaatataatc tcgagctgtg cgaagaaatt agcaaaatga acgagaacct gccgttgtac    240

gcgttcgcta atacgtattc cactctcgat gtaagcctga atgacctgcg tttacagatt    300

agcttctttg aatatgcgct gggtgctgct gaagatattg ctaataagat caagcagacc    360

actgacgaat atatcaacac tattctgcct ccgctgacta aagcactgtt taaatatgtt    420

cgtgaaggta aatatacttt ctgtactcct ggtcacatgg gcggtactgc attccagaaa    480

agcccggtag gtagcctgtt ctatgatttc tttggtccga ataccatgaa atctgatatt    540

tccatttcag tatctgaact gggttctctg ctggatcaca gtggtccaca caaagaagca    600

gaacagtata tcgctcgcgt ctttaacgca gaccgcagct acatggtgac caacggtact    660

tccactgcga acaaaattgt tggtatgtac tctgctccag caggcagcac cattctgatt    720

gaccgtaact gccacaaatc gctgacccac ctgatgatga tgagcgatgt tacgccaatc    780

tatttccgcc cgacccgtaa cgcttacggt attcttggtg gtatcccaca gagtgaattc    840

cagcacgcta ccattgctaa gcgcgtgaaa gaaacaccaa acgcaacctg gccggtacat    900

gctgtaatta ccaactctac ctatgatggt ctgctgtaca acaccgactt catcaagaaa    960

acactggatg tgaaatccat ccactttgac tccgcgtggg tgccttacac caacttctca   1020

ccgatttacg aaggtaaatg cggtatgagc ggtggccgtg tagaagggaa agtgatttac   1080

gaaacccagt ccactcacaa actgctggcg gcgttctctc aggcttccat gatccacgtt   1140

aaaggtgacg taaacgaaga aacctttaac gaagcctaca tgatgcacac caccacttct   1200

ccgcactacg gtatcgtggc gtccactgaa accgctgcgg cgatgatgaa aggcaatgca   1260

ggtaagcgtc tgatcaacgg ttctattgaa cgtgcgatca aattccgtaa agagatcaaa   1320

cgtctgagaa cggaatctga tggctggttc tttgatgtat ggcagccgga tcatatcgat   1380

acgactgaat gctggccgct gcgttctgac agcacctggc acggcttcaa aaacatcgat   1440

aacgagcaca tgtatcttga cccgatcaaa gtcaccctgc tgactccggg gatggaaaaa   1500
```

gacggcacca tgagcgactt tggtattccg gccagcatcg tggcgaaata cctcgacgaa 1560

catggcatcg ttgttgagaa aaccggtccg tataacctgc tgttcctgtt cagcatcggt 1620

atcgataaga ccaaagcact gagcctgctg cgtgctctga ctgactttaa acgtgcgttc 1680

gacctgaacc tgcgtgtgaa aaacatgctg ccgtctctgt atcgtgaaga tcctgaattc 1740

tatgaaaaca tgcgtattca ggaactggct cagaatatcc acaaactgat tgttcaccac 1800

aatctgccgg atctgatgta tcgcgcattt gaagtgctgc cgacgatggt aatgactccg 1860

tatgctgcat tccagaaaga gctgcacggt atgaccgaag aagtttacct cgacgaaatg 1920

gtaggtcgta ttaacgccaa tatgatcctt ccgtacccgc cgggagttcc tctggtaatg 1980

ccgggtgaaa tgatcaccga agaaagccgt ccggttctgg agttcctgca gatgctgtgt 2040

gaaatcggcg ctcactatcc gggctttgaa accgatattc acggtgcata ccgtcaggct 2100

gatggccgct ataccgttaa ggtattgaaa gaagaaagca aaaaataa 2148

<210> 2
<211> 195
<212> DNA
<213> Corynebacterium glutamicum

<400> 2
ttgtggcctt gaaagtgtgc aggatttttg aattctcttt ggagttttcg gcgcgtatgt 60

cagataaaaa ataactgctg gctacaatgg cacgtgaaga acagtatgat aaatggaaat 120

tccagtcatg agagattctt gtggctgagt cccggccctg cctggggcca ccgttaaatc 180

gaagggaatc cgcaa 195

<210> 3
<211> 421
<212> PRT
<213> Corynebacterium glutamicum

<400> 3

Val Ala Leu Val Val Gln Lys Tyr Gly Gly Ser Ser Leu Glu Ser Ala
1 5 10 15

Glu Arg Ile Arg Asn Val Ala Glu Arg Ile Val Ala Thr Lys Lys Ala
20 25 30

Gly Asn Asp Val Val Val Val Cys Ser Ala Met Gly Asp Thr Thr Asp
35 40 45

Glu Leu Leu Glu Leu Ala Ala Ala Val Asn Pro Val Pro Pro Ala Arg
50 55 60

Glu Met Asp Met Leu Leu Thr Ala Gly Glu Arg Ile Ser Asn Ala Leu
65 70 75 80

Val Ala Met Ala Ile Glu Ser Leu Gly Ala Glu Ala Gln Ser Phe Thr
85 90 95

Gly Ser Gln Ala Gly Val Leu Thr Thr Glu Arg His Gly Asn Ala Arg
100 105 110

Ile Val Asp Val Thr Pro Gly Arg Val Arg Glu Ala Leu Asp Glu Gly
115 120 125

Lys Ile Cys Ile Val Ala Gly Phe Gln Gly Val Asn Lys Glu Thr Arg
130 135 140

Asp Val Thr Thr Leu Gly Arg Gly Gly Ser Asp Thr Thr Ala Val Ala
145 150 155 160

Leu Ala Ala Ala Leu Asn Ala Asp Val Cys Glu Ile Tyr Ser Asp Val
165 170 175

Asp Gly Val Tyr Thr Ala Asp Pro Arg Ile Val Pro Asn Ala Gln Lys
180 185 190

Leu Glu Lys Leu Ser Phe Glu Glu Met Leu Glu Leu Ala Ala Val Gly
195 200 205

Ser Lys Ile Leu Val Leu Arg Ser Val Glu Tyr Ala Arg Ala Phe Asn
210 215 220

Val Pro Leu Arg Val Arg Ser Ser Tyr Ser Asn Asp Pro Gly Thr Leu
225 230 235 240

Ile Ala Gly Ser Met Glu Asp Ile Pro Val Glu Glu Ala Val Leu Thr
245 250 255

Gly Val Ala Thr Asp Lys Ser Glu Ala Lys Val Thr Val Leu Gly Ile
260 265 270

Ser Asp Lys Pro Gly Glu Ala Ala Lys Val Phe Arg Ala Leu Ala Asp
275 280 285

Ala Glu Ile Asn Ile Asp Met Val Leu Gln Asn Val Ser Ser Val Glu
290 295 300

Asp Gly Thr Thr Asp Ile Thr Phe Thr Cys Pro Arg Ser Asp Gly Arg
305 310 315 320

Arg Ala Met Glu Ile Leu Lys Lys Leu Gln Val Gln Gly Asn Trp Thr
325 330 335

Asn Val Leu Tyr Asp Asp Gln Val Gly Lys Val Ser Leu Val Gly Ala
340 345 350

Gly Met Lys Ser His Pro Gly Val Thr Ala Glu Phe Met Glu Ala Leu
355 360 365

Arg Asp Val Asn Val Asn Ile Glu Leu Ile Ser Thr Ser Glu Ile Arg
    370                 375                 380

Ile Ser Val Leu Ile Arg Glu Asp Asp Leu Asp Ala Ala Ala Arg Ala
385                 390                 395                 400

Leu His Glu Gln Phe Gln Leu Gly Gly Glu Asp Glu Ala Val Val Tyr
                405                 410                 415

Ala Gly Thr Gly Arg
            420

<210> 4
<211> 29
<212> DNA
<213> Artificial

<220>
<223> primer

<400> 4
atagcatgcg tggccctggt cgtacagaa 29

<210> 5
<211> 27
<212> DNA
<213> Artificial

<220>
<223> primer

<400> 5
ataggatcct tagcgtccgg tgcctgc 27

<210> 6
<211> 26
<212> DNA
<213> Artificial

<220>
<223> primer

<400> 6
gcaccaccga catcatcttc acctgc 26

<210> 7
<211> 25
<212> DNA
<213> Artificial

<220>
<223> primer

<400> 7
gagggcaggt gaagatgatg tcggt 25

<210> 8
<211> 29
<212> DNA

<213> Artificial

<220>
<223> primer

<400> 8
gaagagctcg atccttttta acccatcac 29

<210> 9
<211> 31
<212> DNA
<213> Artificial

<220>
<223> primer

<400> 9
gaagagctcc aagtcgataa acagcaatat t 31

<210> 10
<211> 29
<212> DNA
<213> Artificial

<220>
<223> primer

<400> 10
attggatccc ctgaatcgcc ccatcatcc 29

<210> 11
<211> 35
<212> DNA
<213> Artificial

<220>
<223> primer

<400> 11
ataggtaccc catggcaccc cttgtattac tgttt 35

<210> 12
<211> 29
<212> DNA
<213> Artificial

<220>
<223> primer

<400> 12
ataccatgga cgttattgca atattgaat 29

<210> 13
<211> 37
<212> DNA
<213> Artificial

<220>
<223> primer

<400> 13
atagagctct tattttttgc tttcttcttt caatacc 37

<210> 14
<211> 28
<212> DNA
<213> Artificial

<220>
<223> primer

<400> 14
atagcatgca tgacctcagc atctgccc 28

<210> 15
<211> 27
<212> DNA
<213> Artificial

<220>
<223> primer

<400> 15
ataggatcct tagtcccttt cgaggcg 27

<210> 16
<211> 29
<212> DNA
<213> Artificial

<220>
<223> primer

<400> 16
gaaagcgctc ctgaatcgcc ccatcatcc 29

<210> 17
<211> 30
<212> DNA
<213> Artificial

<220>
<223> primer

<400> 17
gaaagcgctt tattttttgc tttcttcttt 30

<210> 18
<211> 29
<212> DNA
<213> Artificial

<220>
<223> primer

<400> 18
gaagaattct aaacctcagc atctgcccc 29

<210> 19
<211> 30
<212> DNA
<213> Artificial

<220>
<223> primer

<400> 19
gaaccgcggt tattttttgc tttcttcttt 30

<210> 20
<211> 1098
<212> DNA
<213> Corynebacterium glutamicum

<400> 20
atgccgttga ctccagctga tgtgcataac gtcgctttta ataagccgcc tatcggcaag 60

cgtggctaca acgaagacga ggttgatcag ttcctagatc tcgttgagga cgccctcgtt 120

cagttccaag aggaaaacga agacctaaag cagcaggtcg aagagctaga ggcgcaggtt 180

gccggtggta cttcttccgc tgctagttcc tcaactgcag gtgcagccac agctgcagct 240

tccaagtctg ttgacgaggc agcgctgcgc aaggaaatcg aagagaagct gcgctccgaa 300

tacgcatcca agctcgatga tgcctccaag gccgctcaga aggctcaaaa cgatgcgaag 360

tccgctcaag atcagctaca gcgtgcacaa gctgacgcaa aggcagctcg cgacgaagct 420

gaaaaggcca aggctgaagc taagtcagca gcatcctcca gcaccactaa ggcagcagcg 480

gttggcgctg tcggcgctgg caccggagca gcagttgcta caggtgctgc aaatgtggac 540

acccacatgc aggcagcgaa ggttctggga ctcgcacagg aaatggcaga ccgcctgacc 600

tcagaggctc gctccgaatc caagtccatg ctggacgagg ctcgcgaagc agcagagaag 660

cagatcgagg aagcaaacag cacctccaac cgcactctgg aagatgctcg cgcaaacgct 720

gagaagcaga tcgctgaagc gcagaaccgc gctgacactc tggtcaacga agctgacgct 780

aaggctaaga acctggtttc cgaagccgag aagaagtccg cagccaccct ggccgcatcc 840

acctctcgtg cagaagctca gatccgtcaa gccgaggaca aggcaaacgc cctccaggca 900

gacgcagagc gcaagcacac cgaaaccatg gctgcagtca aggaacagca gaatgctctg 960

gagacccgca tcgcggaact gcagaccttc gagcgtgagt accgcacccg tctgaagtcc 1020

ctcctcgagg gccagctgga agaactcaac gcacgtggct cctctgcacc aaccaacaac 1080

aagccatctg gtgagtaa 1098

<210> 21
<211> 30
<212> DNA
<213> Artificial

<220>
<223> primer

<400> 21
atgcggatcc ttgtggcctt gaaagtgtgc 30

<210> 22
<211> 30
<212> DNA
<213> Artificial

<220>

<223> primer

<400> 22
atgcccatgg ttgcggattc ccttcgattt 30

<210> 23
<211> 29
<212> DNA
<213> Artificial

<220>
<223> primer

<400> 23
ataccatgga cgttattgca atattgaat 29

<210> 24
<211> 29
<212> DNA
<213> Artificial

<220>
<223> primer

<400> 24
atagagctct tattttttgc tttcttctt 29

<210> 25
<211> 29
<212> DNA
<213> Artificial

<220>
<223> primer

<400> 25
ataagatctt tctccgaccg cgtcaaccg 29

<210> 26
<211> 29
<212> DNA
<213> Artificial

<220>
<223> primer

<400> 26
ataggatccg agttcgagta gttctgcgg 29

<210> 27
<211> 29
<212> DNA
<213> Artificial

<220>
<223> primer

<400> 27
atatctagaa agtttctctc cttagctat 29

<210> 28
<211> 29

<212> DNA
<213> Artificial

<220>
<223> primer

<400> 28
atagcatgcc aaaatacccg aagcaacac 29

**Description**

TECHNICAL FIELD

The present invention relates to a method for producing 1,5-pentanediamine by the fermentation method.

BACKGROUND ART

Polyamide (PA) is a group of important polymers that are used as raw materials for a series of special plastics to be used in the automobile industry, sports industry and lifestyle industry, and diamines are important raw material monomer components for the polyamides. Diamines are condensed with dicarboxylic acids to form various polymers, and the properties of the polymers vary depending on the chain lengths of the diamines and the dicarboxylic acids.

Conventionally, diamines are chemically produced from materials derived from petroleum via dicarboxylic acids at an intermediate stage, or produced by chemical decarboxylation reaction of amino acids (Non-patent Document 1). In consideration of sharp rise in oil prices, the production methods are preferably immediately switched to methods based on biotechnological processes such as fermentation, wherein renewable resources are utilized to synthesize diamines..

In view of this, recent interest has focused on methods for producing 1,5-pentanediamine; which is a diamine having 5 carbon atoms, by fermentation. 1,5-Pentanediamine is also called cadaverine, and is a compound which can be used as raw material monomers for polyamides. 1,5-Pentanediamine is a polyamine which ubiquitously exists in the living body and its biosynthetic system is being elucidated (see Non-patent Document 2). In a part of its biosynthetic pathway, L-lysine decarboxylase (hereinafter referred to as LDC), which catalyzes decarboxylation of L-lysine, is known to be involved.

Conventional methods for producing 1,5-pentanediamine by fermentation are based on introduction of an LDC gene to a microorganism, and examples of the methods include a production method by fermentation in a recombinant *E. coli* (see Patent Document 1), method by further enhancing the lysine production capacity of a coryneform bacterium, which is a lysine-producing microorganism (see Patent Document 2), method by blocking the 1,5-pentanediamine degradation system (see Patent Document 3), and method by supplying lysine decarboxylase by an autonomously replicating vector (see Non-patent Document 3).

However, there are many problems to be solved in the methods for producing 1,5-pentanediamine by fermentation, and examples of such problems include by-production of lysine in cases of fermentation using a microorganism prepared by introduction of an LDC gene to a coryneform bacterium, which is a lysine-producing microorganism (see Non-patent Document 4). That is, there has been a problem that the culture supernatant contains a large amount of unreacted lysine, which is the immediate precursor of 1,5-pentanediamine in its biosynthesis. Such by-production of lysine prevents improvement of the fermentation yield of 1,5-pentanediamine even with successful production of its precursor, which has been economically problematic. Further, since, in Patent Document 3 and Non-patent Document 3, the LDC gene is supplied by an autonomously replicating vector, the culture needs to be carried out in the presence of an expensive antibiotic or the like. Therefore, use of a microorganism having an LDC gene in its chromosome is demanded for low-cost fermentation production of 1,5-pentanediamine on an industrial scale.

PRIOR ART DOCUMENTS

[Patent Documents]

Patent Document 1: JP 2002-223770 A
Patent Document 2: JP 2004-222569 A
Patent Document 3: Japanese Translated PCT Patent Application Laid-open No. 2009-531042

[Non-patent Documents]

Non-patent Document 1: Suyama and Kaneo. Yakugaku Zasshi (1965), Vol. 85, pp. 513-533.
Non-patent Document 2: Celia White Tabor and a colleague. Microbiological Reviews (1985), Vol. 49, pp. 81-99.
Non-patent Document 3: Tateno et al. Appl Microbiol Biotechnol (2009), 81(1), pp. 115-21.
Non-patent Document 4: Mimitsuka et al. Biosci Biotechnol Biochem (2007), 71(9), pp. 2130-5.

SUMMARY OF THE INVENTION

PROBLEMS TO BE SOLVED BY THE INVENTION

An object of the present invention is to provide a method for producing 1,5-pentanediamine by fermentation using a microorganism which has an LDC gene in its chromosome and which shows suppressed by-production of L-lysine.

MEANS FOR SOLVING THE PROBLEMS

The present inventors intensively studied a method for producing 1,5-pentanediamine by the fermentation method while suppressing by-production of L-lysine, and, as a result, discovered that by-production of L-lysine can be suppressed by using a coryneform bacterium which has an LDC gene in its chromosome and which maintains a specific activity of lysine decarboxylase of not less than 50 mU/mg protein during culturing for fermentation production of 1,5-pentanediamine, thereby completing the present invention. That is, the present invention is constituted by (1) to (12) below.

(1) A method for producing 1,5-pentanediamine using a coryneform bacterium having a gene encoding lysine decarboxylase in its chromosome, which coryneform bacterium maintains lysine decarboxylase activity of not less than 50 mU/mg protein during culturing.
(2) A method for producing 1,5-pentanediamine using a coryneform bacterium having a gene encoding lysine decarboxylase in its chromosome, the gene encoding lysine decarboxylase being linked downstream of a promoter that functions during the logarithmic growth phase.
(3) The method according to (2), wherein the promoter is the divIVA gene promoter.
(4) The method according to (2) or (3), wherein the promoter is a promoter selected from (A) to (D) below:

(A) a promoter having the base sequence shown in SEQ ID NO:2;
(B) a promoter having the same base sequence as the base sequence shown in SEQ ID NO:2 except that one or several bases are substituted, deleted, inserted and/or added;
(C) a promoter that entirely or partially hybridizes under stringent conditions with the promoter having the base sequence shown in SEQ ID NO:2 or the complementary strand thereof; and
(D) a promoter having a base sequence having a sequence identity of not less than 80% to the base sequence shown in SEQ ID NO:2.

(5) The method according to any of (1) to (4), wherein the gene encoding lysine decarboxylase is a gene derived from *E. coli.*
(6) The method according to any of (1) to (5), wherein the gene encoding lysine decarboxylase is a gene selected from (A) to (D) below and encodes a protein having lysine decarboxylase activity:

(A) a gene having the base sequence shown in SEQ ID NO:1;
(B) a gene having the same base sequence as the base sequence shown in SEQ ID NO:1 except that one or several bases are substituted, deleted, inserted and/or added;
(C) a gene that entirely or partially hybridizes under stringent conditions with a gene having the base sequence shown in SEQ ID NO:1 1 or the complementary strand thereof; and
(D) a gene having a base sequence having a sequence identity of not less than 80% to the base sequence shown in SEQ ID NO:1.

(7) The method according to any of (1) to (6), wherein the coryneform bacterium is a coryneform bacterium having improved L-lysine productivity.
(8) The method according to any of (1) to (7), wherein the coryneform bacterium has a mutant-type aspartate kinase having the same amino acid sequence as shown in SEQ ID NO:3 except that the 311th amino acid residue is substituted by an amino acid other than threonine.
(9) The method according to any of (1) to (8), wherein the coryneform bacterium has decreased homoserine dehydrogenase activity or is deficient for homoserine dehydrogenase activity.
(10) The method according to (9), wherein the coryneform bacterium is deficient for homoserine dehydrogenase activity because of insertional mutagenesis.
(11) The method according to any of (1) to (10), wherein the coryneform bacterium is a bacterium belonging to the genus *Corynebacterium.*
(12) The method according to (11), wherein the bacterium belonging to the genus *Corynebacterium* is *Corynebac-*

*terium glutamicum.*

BRIEF DESCRIPTION OF THE DRAWINGS

Fig. 1 is a diagram showing changes in the glucose concentration, lysine concentration and 1,5-pentanediamine (1,5-PD) concentration in the culture supernatant with time during culturing of the coryneform bacterium CG4541 strain prepared in Example of the present invention.

Fig. 2 is a diagram showing changes in the glucose concentration, lysine concentration and 1,5-pentanediamine (1,5-PD) concentration in the culture supernatant with time during culturing of the coryneform bacterium TM4552 strain prepared in Comparative Example of the present invention.

Fig. 3 is a diagram showing changes in the specific activity of lysine decarboxylase with time during culturing of the coryneform bacterium CG4541 strain prepared in Example of the present invention.

BEST MODE FOR CARRYING OUT THE INVENTION

Embodiments of the present invention are described below in more detail.

The lysine decarboxylase (LDC) in the present invention means an enzyme that can convert L-lysine as a substrate to 1,5-pentanediamine by decarboxylation reaction, and may also have another/other enzymatic action(s).

The LDC used in the present invention is not restricted, and is preferably derived from *Bacillus halodurans, Bacillus subtilis, Escherichia coli, Selenomonas ruminamtium, Vibrio cholerae, Vibrio parahaemolyticus, Streptomyces coelicolor, Streptomyces pilosus, Eikenella corrodens, Eubacterium acidaminophilum, Salmonella typhimurium, Hafnia alvei, Neisseria meningitidis, Thermoplasma acidophilum* or *Pyrococcus abyssi.* The LDC is more preferably derived from E. *coli.*

Specific examples of the gene encoding lysine decarboxylase used in the present invention include the genes encoding lysine decarboxylase derived from the above-described organisms. The base sequence of the gene may be redesigned depending on the codon usage of the microorganism used. The base sequences encoding lysine decarboxylase derived from the above-described organisms are registered in a database (GenBank). Among these, the gene derived from *E. coli,* which has the base sequence shown in SEQ ID NO:1, is preferred.

The specific activity of LDC is described below. When the amount of LDC that produces 1 μmol of 1,5-pentanediamine per minute is defined as 1 unit (U), the LDC activity is represented by Equation 1 below.

[Equation 1]

$$\text{Activity [U]} = \frac{\text{Amount of 1,5-pentanediamine produced } [\mu g]}{\text{MW (102. 18)} [\mu g / \mu mol] \times \text{Reaction time [min]}} \quad \text{(Equation 1)}$$

By this, the activity per unit amount of protein, that is, the specific activity, of LDC can be calculated according to Equation 2 below.

[Equation 2]

$$\text{Specific activity [U/mg]} = \frac{\text{Activity [U]}}{\text{Amount of protein [mg]}} \quad \text{(Equation 2)}$$

The specific activity of LDC in the present invention means the specific activity per total protein of the coryneform bacterium, and can be calculated by measuring the specific activity using total protein extracted from the coryneform bacterium. The operation of measurement is described in the Example below more specifically.

The method of extraction of protein from the coryneform bacterium is not restricted, and may be a method wherein bacterial cells are homogenized by sonication, method wherein bacterial cells are homogenized using glass beads with stirring by vortexing or the like, or method wherein bacterial cells are homogenized under high pressure; or a combination of these methods. The method is preferably a method using glass beads.

The coryneform bacterium having a gene encoding lysine decarboxylase in its chromosome in the present invention has an LDC specific activity of not less than 50 mU/mg protein, preferably has an LDC specific activity of not less than 80 mU/mg protein, more preferably has an LDC specific activity of not less than 180 mU/mg protein. Further, as described in the Example below, even in cases where the bacterium has an LDC specific activity of not less than 50mU/mg protein at a certain time point during the culturing, a decrease in the specific activity after the time point causes accumulation of the precursor L-lysine. Therefore, the coryneform bacterium used in the present invention maintains the above LDC specific activity throughout the culture period.

The term "throughout the culture period" means the period which begins when the coryneform bacterium is inoculated to the fermentation medium and continues while the substrate sugar is being consumed, and the period can be confirmed by measuring the specific activity at an arbitrary time point during the period. The culture period after the beginning of culturing, during which the LDC specific activity is maintained, is preferably not less than 5 hours, more preferably not less than 10 hours, still more preferably not less than 20 hours. The upper limit of the culture period during which the above-described specific activity can be maintained is not restricted.

The method for giving the LDC specific activity as described above is not restricted, and examples of the method include a method wherein the copy number of the gene encoding the lysine decarboxylase to be introduced into the chromosome of the coryneform bacterium is increased, method wherein a strong promoter is used as the promoter of the gene encoding the lysine decarboxylase to be introduced into the chromosome, and method wherein a gene encoding a lysine decarboxylase having an enhanced enzyme activity is employed. Any of these methods may be preferably used, and the method is more preferably the one wherein a strong promoter is used as the promoter of the gene encoding the lysine decarboxylase to be introduced into the chromosome.

The promoter used in the present invention is not restricted, and a promoter that can function in a coryneform bacterium may be generally used. Further, the promoter may be one derived from a different species. Preferred examples of the promoter include:

promoters involved in various amino acid biosynthetic systems, such as the promoters of the glutamate dehydrogenase gene involved in the glutamic acid biosynthetic system; glutamine synthetase gene involved in the glutamine biosynthetic system; aspartokinase gene involved in the lysine biosynthetic system; homoserine dehydrogenase gene involved in the threonine biosynthetic system; acetohydroxy acid synthetase gene involved in the isoleucine and valine biosynthetic systems; 2-isopropyl malic acid synthetase gene involved in the leucine biosynthetic system; glutamate kinase gene involved in the proline and arginine biosynthetic systems; phosphoribosyl-ATP pyrophosphorylase gene involved in the histidine biosynthetic system; and deoxy arabino heptulosonate phosphate (DAHP) synthase gene involved in the biosynthetic systems of aromatic amino acids such as tryptophan, tyrosine and phenylalanine;
promoters involved in the biosynthetic systems of nucleic acids such as inosinic acid and guanylic acid, including the promoters of the phosphoribosyl pyrophosphate (PRPP) amidotransferase gene, inosinic acid dehydrogenase gene and guanylic acid synthetase gene;
promoters of genes involved in cell division, such as the promoters of the divIVA gene and the like;
strong promoters such as the tac promoter, trc promoter and HCE promoter; and
promoters that function during the logarithmic growth phase (exponential phase).

The promoter is more preferably one that functions during the logarithmic growth phase. Specific examples of the promoter that functions during the logarithmic growth phase include the promoters of genes such as the divIVA, gap, ldhA, fda, glyA, cysK, aroF, gpmA, eno, fumC, pfk, sdhA, mdh, argF, proA, proC, aceE, serA, metE, nifS1, tpi, aceD, cysD, sdhB and pck genes. Among these, the promoter of the divIA gene is preferred, and, more specifically, the promoter having the base sequence shown in SEQ ID NO:2 is especially preferred.

Examples of the promoter sequence and the base sequence of the gene encoding lysine decarboxylase also include base sequences which are the same as their respective sequences except that one or several amino acids are substituted, deleted, inserted and/or added, as long as their functions are maintained. The term "several" herein means normally about 1 to 40, preferably about 1 to 30, more preferably about 1 to 20, still more preferably about 1 to 9, still more preferably about 1 to 5. Further, examples of the promoter sequence and the base sequence of the gene encoding lysine decarboxylase include base sequences that entirely or partially hybridize with those base sequences or with the complementary strands thereof under stringent conditions, as long as their functions are maintained. The term "polynucleotide that hybridizes under stringent conditions" herein means, for example, a base sequence that hybridizes with a probe(s) having one or more base sequences each having at least 20, preferably 25, more preferably at least 30

continuous sequences arbitrarily selected from the original base sequence, when a known hybridization technique (Current Protocols I Molecular Biology edit. Ausbel et al., (1987) Publish. John Wily & Sons Section 6.3-6.4) or the like is applied. The stringent conditions herein can be achieved, for example, by performing hybridization in the presence of 50% formamide at a temperature of 37°C, at 42°C for more stringent conditions, or at 65°C for even more stringent conditions, followed by washing with 0.1 × to 2×SSC solution (composition of × 1 SSC solution: 150 mM sodium chloride, 15 mM sodium citrate). The promoter sequence and the base sequence encoding lysine decarboxylase may be sequences having sequence identities of normally not less than 80%, preferably not less than 90%, more preferably not less than 95%, still more preferably not less than 99%. The sequence identity herein means a value calculated by aligning two base sequences such that the number of matched bases is maximum (by insertion of a gap(s), as required) and dividing the number of matched bases by the number of bases of the full-length sequence (in cases where the total number of bases is different between the two sequences, the number of bases of the longer sequence). Such calculation of the homology can be easily carried out using well-known software such as BLAST.

Such a promoter sequence and base sequence encoding lysine decarboxylase can also be obtained from an organism other than coryneform bacteria, and can also be obtained by subjecting a coryneform bacterium-derived base sequence to in vitro mutagenesis or site-directed mutagenesis.

The method of introduction of the promoter sequence and base sequence encoding lysine decarboxylase to the coryneform bacterium is not restricted, and the sequences may be introduced by electroporation (Bio/Technology, (1989), 7, 1067-1070).

Coryneform bacteria are aerobic gram-positive bacilli, and also include bacteria which had previously been classified in the genus *Brevibacterium* but have now been integrated into the genus *Corynebacterium* (Int. J. Syst., Bacteriol., (1981) 41, p. 225). Coryneform bacteria also include the bacteria belonging to the genus *Brevibacterium,* which is very close to the genus *Corynebacterium.* Examples of such coryneform bacteria include *Corynebacterium acetoacidophylum, Corynebacterium acetoglutamicum, Corynebacterium alkanolyticum, Corynebacterium callunae, Corynebacterium glutamicum, Corynebacterium lilium, Corynebacterium mellassecola, Corynebacterium thermoaminogenes, Corynebacterium efficient, Corynebacterium herculis, Brevibacterium divaricatum, Brevibacterium flavum, Brevibacterium immariophilum, Brevibacterium lactofermentum, Brevibacterium roseum, Brevibacterium saccharolyticum, Brevibacterium thiogenitalis, Corynebacterium ammoniagenes, Brevibacterium album, Brevibacterium cerinum* and *Microbacterium ammoniaphilum.*

Specific examples of strains of the respective coryneform bacteria include *Corynebacterium acetoacidophylum* ATCC13870, *Corynebacterium acetoglutamicum* ATCC15806, *Corynebacterium alkanolyticum* ATCC21511, *Corynebacterium callunae* ATCC15991, *Corynebacterium glutamicum* ATCC13020, ATCC13020 and ATCC13060, *Corynebacterium lilium* ATCC15990, *Corynebacterium mellassecola* ATCC17965, *Corynebacterium efficient* AJ12340 (accession No. FERM BP-1539), *Corynebacterium herculis* ATCC13868, *Brevibacterium divaricatum* ATCC14020, *Brevibacterium flavum* ATCC13826, ATCC14067 and AJ12418 (accession No. FERM BP-2205), *Brevibacterium immariophilum* ATCC14068, *Brevibacterium lactofermentum* ATCC13869, *Brevibacterium roseum* ATCC13825, *Brevibacterium saccharolyticum* ATCC14066, *Brevibacterium thiogenitalis* ATCC19240, *Corynebacterium ammoniagenes* ATCC6871 and ATCC6872, *Brevibacterium album* ATCC15111, *Brevibacterium cerinum* ATCC15112 and *Microbacterium ammoniaphilum* ATCC15354.

The above coryneform bacteria are available from, for example, American Type Culture Collection. That is, a corresponding accession number is given to each strain and described in the catalogue of American Type Culture Collection, and each strain can be obtained by reference to this number.

The coryneform bacterium to be used in the method of the present invention for producing 1,5-pentanediamine, which bacterium has a gene encoding lysine decarboxylase in the chromosome, is preferably a coryneform bacterium having improved productivity of L-lysine, which is a precursor of 1,5-pentanediamine. The method for improving the L-lysine productivity of the coryneform bacterium is not restricted, and a known method may be used. Examples of the method include: a method wherein, as described in JP 2004-222569 A, a strain resistant to S-aminoethylcysteine (AEC) is obtained; and a method wherein, as described in Journal of industrial Microbiol Biotechnol (2006, 33(7) 610-5) and Japanese Translated PCT Patent Application Laid-open No. 2009-531042, the L-lysine productivity is improved by genome breeding. Both of these methods may be preferably employed.

In a preferred mode of the Coryneform bacterium of the present invention having improved L-lysine productivity, the bacterium preferably has aspartate kinase whose feedback inhibition by L-lysine is canceled, and the bacterium more preferably has a mutant aspartate kinase having the same amino acid sequence as described in SEQ ID NO:3 except that the 311 th amino acid residue is replaced with an amino acid other than threonine.

In another preferred mode of the coryneform bacterium of the present invention having improved L-lysine productivity, the bacterium preferably has decreased homoserine dehydrogenase activity or is deficient for the homoserine dehydrogenase activity. The bacterium is more preferably deficient for the homoserine dehydrogenase activity because of destruction or disruption of the homoserine dehydrogenase gene by insertional mutagenesis.

Examples of the culture method which may be used include batch culture, fed-batch culture and continuous

culture. In cases of continuous culture, continuous culture described in JP 2008-104453 A or the like is preferably carried out.

As a culture medium, a normal nutrient medium comprising a carbon source, nitrogen source, inorganic salt and/or the like may be used. Examples of the carbon source which may be used include saccharides such as glucose, fructose, sucrose, maltose and starch hydrolysates; alcohols such as ethanol; and organic acids such as acetic acid, lactic acid and succinic acid. Examples of the nitrogen source which may be used include ammonia; inorganic and organic ammonium salts such as ammonium chloride, ammonium sulfate, ammonium carbonate and ammonium acetate; nitrogen-containing organic compounds such as urea; and nitrogen-containing organic substances such as meat extracts, yeast extracts, corn steep liquor and soybean hydrolysates. Examples of the inorganic salt which may be used include potassium dihydrogen phosphate, dipotassium hydrogen phosphate, ammonium sulfate, sodium chloride, magnesium sulfate and calcium carbonate. Further, as required, micronutrients such as biotin, thiamine, vitamin B6 and the like may be added. Medium additives such as meat extracts, yeast extracts, corn steep liquor and casamino acids may be used as alternatives to these micronutrients.

The culture conditions are not restricted, and the culture is carried out under aerobic conditions, for example, with shaking or by deep aeration stirring culture. The culture temperature is generally 25°C to 42°C, preferably 28°C to 38°C. The culture period is normally 1 day to 10 days.

For adjusting the culture pH, ammonia, hydrochloric acid or dicarboxylic acid is preferably used, and dicarboxylic acid is more preferably used. It is preferred to use the neutralizer to control the culture pH to 5 to 8, more preferably 6.5 to 7.5. The state of the neutralizer is not restricted, and the neutralizer may be used as a gas, liquid, solid or an aqueous solution. The neutralizer is especially preferably an aqueous solution.

The dicarboxylic acid to be preferably used as the neutralizer is not restricted, and the dicarboxylic acid is preferably a dicarboxylic acid having substantially no functional group other than the above-described two carboxylic groups. The functional group herein means a reactive group which reacts, during polyamide polymerization reaction (under reaction conditions wherein, for example, the reaction temperature is 250 to 270°C, the pressure is 10 to 20 $kg/cm^2$, and the reaction time is 1 to 5 hour(s)), with an amino group or carboxyl group to cause branching of the polymer or reduction in the degree of crystallinity of the polymer (to a degree of crystallinity of not more than 80%). Examples of the functional group include the amino group and carboxyl group, and other examples of the functional group include acidic groups (e.g., the sulfonic acid group, phosphate group and phenolic hydroxyl group), basic groups (e.g., the hydrazino group), protonic polar groups (e.g., the hydroxyl group), cleavable groups (e.g., the epoxy group and peroxide group) and other highly reactive groups (e.g., isocyanate group). On the other hand, halogen substituents, aromatic substituents, ether groups, ester groups, amide groups and the like are not included in the functional group in the present description since their reactivity is low.

The dicarboxylic acid is more preferably a dicarboxylic acid represented by General Formula (1), (2) or (3) below.

$$HOOC\text{-}(CH_2)_m\text{-}COOH \qquad (1)$$

(wherein m = 0 to 16)

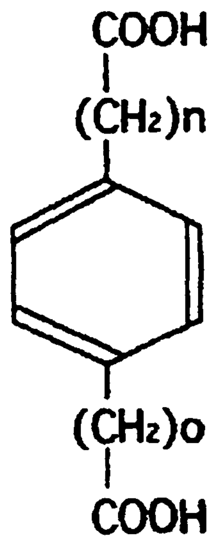

(2)

(wherein n, o = 0 to 16)

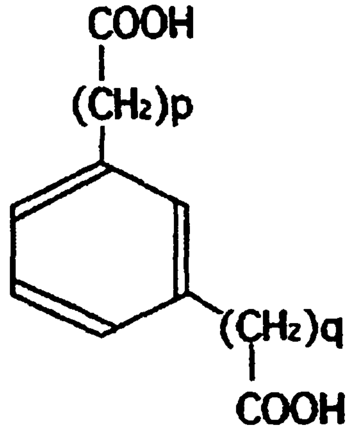

(3)

(wherein p, q = 0 to 16)

The dicarboxylic acid is still more preferably adipic acid, sebacic acid, 1,12-dodecanedicarboxylic acid, succinic acid, isophthalic acid or terephthalic acid.

1,5-Pentanediamine in the culture liquid exists in the free state or as a salt of 1,5-pentanediamine. In the method for collecting 1,5-pentanediamine from the culture liquid, the microorganism is first removed from the culture liquid. In this step, the bacterial cells may be separated from the culture supernatant after the growth of the microorganism and production of 1,5-pentanediamine as a result of sufficient progress of fermentation (the bacterial cells may be separated by removal by precipitation, centrifugation or membrane filtration), or the bacterial cells may be separated, retained or immobilized on a carrier or the like from the beginning. For collecting 1,5-pentanediamine from the culture liquid from which the microorganism was removed and which contains 1,5-pentanediamine, 1,5-pentanediamine dicarboxylate may be collected by crystallization as described in JP 2009-207495 A. Alternatively, 1,5-pentanediamine may be purified and collected using an NF membrane as described in JP 2009-29872 A. Alternatively, 1,5-pentanediamine may be collected by extraction with a polar organic solvent followed by distillation as described in JP 2009-28045 A.

EXAMPLES

Modes of the present invention will now be described below by way of Examples, but these are merely examples of the present invention and do not limit the present invention.

Method of Analysis of Concentrations of 1,5-Pentanediamine and L-lysine by HPLC

Column used: CAPCELL PAK C18 (Shiseido)
Mobile phase: 0.1 % (w/w) aqueous phosphate solution:acetonitrile = 4.5:5.5
Detection: UV 360 nm

Sample pretreatment: To 25 μl of the sample to be analyzed, 25 μl of 1,4-diaminobutane (0.03 M) as an internal standard, 150 μl of sodium hydrogen carbonate (0.075 M) and a solution of 2,4-dinitrofluorobenzene (0.2 M) in ethanol were added, and the resulting mixture was mixed, followed by being incubated at 37°C for 1 hour. A 50-μl aliquot of the above reaction solution was dissolved in 1 ml of acetonitrile, and resulting the solution was centrifuged at 10,000 rpm for 5 minutes, followed by subjecting 10 μl of the supernatant to HPLC analysis.

Example 1, Comparative Example 1

(1) Preparation of *Corynebacterium glutamicum* Capable of Fermentation Production of L-Lysine

In order to prepare *Corynebacterium glutamicum* capable of synthesizing L-lysine as a precursor of 1,5-pentanediamine, an L-lysine fermentation bacterium was prepared by introduction of an effective mutation to aspartokinase (AK). Referring to the method described in Apppl. Microbiol. Biotechnol., (2002), 58, pp. 217-223, the *Corynebacterium*

*glutamicum* AK-1 strain (hereinafter referred to as the AK-1 strain) was prepared. More specifically, the operation was carried out as follows.

PCR was carried out using as an amplification template a genomic DNA solution prepared from the *Corynebacterium glutamicum* ATCC13032 strain according to a conventional method, and oligonucleotides (SEQ ID NOs:4 and 5) designed by reference to a base sequence of the AK gene (Accession No. BA000036) registered in a database (GenBank) as a primer set, and the obtained product was subjected to electrophoresis in 1% agarose gel, followed by excising the DNA fragment of about 1.3 kb containing the AK gene from the gel and purifying the fragment using Gene Clean Kit. This fragment was digested with *Bam*HI and *Sph*I*,* and the resulting *Bam*HI-*Sph*I fragment of about 1.3 kb was ligated to the *Bam*HI/*Sph*I gap in pUC19 that had been preliminarily digested with *Bam*HI and *Sph*I*.* The obtained plasmid was designated pTM47. The obtained AK gene was confirmed to have the same gene sequence as the one registered in the database by sequencing.

In order to mutate acc (Thr) at positions 931 to 933 in the cloned AK gene to atg (Ile), QuickChange Multi Site-Directed Mutagenesis Kit (manufactured by Stratagene) was used. Details of the experiment were carried out according to the instructions attached to the product. Using pAK1 as an amplification template and the oligonucleotides having the base sequences shown in SEQ ID NOs:6 and 7 as a primer set, the entire sequence of pAK1 was amplified. After treating this PCR product with *Dpn*I*,* the *E. coli* JM109 strain was transformed. After extracting the plasmid, the AK gene sequence was analyzed, and it was revealed that the plasmid wherein the mutation of interest was introduced could be obtained. This plasmid was designated pTM49-1. The thus obtained pTM49-1 was digested with *Sph*I and *Bam*HI*,* and the part containing the AK gene (about 1.3 kb) was obtained by gel extraction, to purify the AK gene to which the mutation was introduced.

PCR was carried out using bacterial cells of the *Bacillus subtilis* IFO13719 strain as a template and the base sequences shown in SEQ ID NOs:8 and 9 as a primer set, to amplify the sacB gene. The obtained PCR product was digested with *Sac*I, and ligated to pHSG298 (a commercially available pUC-type plasmid having as a selection marker a kanamycin resistance gene, manufactured by Takara Bio Inc.) which had been preliminarily similarly digested with *Sac*I. The obtained plasmid was designated pTM38. Subsequently, the above-described fragment containing the AK gene was ligated to pTM38 digested with *Sph*I and *Bam*HI*.* The thus prepared plasmid having the sacB gene and the mutant AK gene was designated pTM52.

pTM52 prepared as described above was introduced to the ATCC13032 strain by electroporation [FEMS Microbiology Letters, 65, p. 299 (1989)], and transformants were selected on an agar medium containing LB (tryptone (10 g/l) (manufactured by Bacto), yeast extract (5 g/l) (manufactured by Bacto) and sodium chloride (10 g/l)) supplemented with kanamycin (25 $\mu$g/ml). Thereafter, the selected kanamycin-resistant coryneform cells were cultured on a medium supplemented with sucrose, and sucrose-resistant coryneform cells were selected by double crossing-over. Among the thus selected transformants, a strain that can grow on a minimum medium supplemented with 20 mM S-aminoethylcysteine (AEC) was selected, and a genomic DNA solution was prepared therefrom according to a conventional method. PCR was carried out using this genomic DNA as a template and the oligonucleotides (SEQ ID NOs:4 and 5) as a primer set, and the AK gene sequence was analyzed. As a result, it could be confirmed that the sequence at positions 931 to 933 was replaced with atg. Feedback inhibition of aspartokinase by lysine and threonine is canceled in the thus prepared AK-1 strain. Therefore, L-lysine can be synthesized by culturing.

Subsequently, the obtained AK-1 strain was subjected to genetic recombination to prepare a coryneform bacterium having a single copy of E. *coli*-derived LDC gene in the chromosome and a coryneform bacterium having two copies of E. coli-derived LDC gene in the chromosome by the following methods.

(2) Preparation of Coryneform Bacterium Wherein Single Copy of LDC Gene is Introduced to Chromosome (Comparative Example 1)

In order to modify the AK-1 strain into a 1,5-pentanediamine fermentation bacterium, a plasmid pTM45 for introduction of an *E. coli*-derived LDC gene into the homoserine dehydrogenase locus was prepared. More specifically, this operation was carried out as follows.

PCR was carried out using pHSG298 as a template and the oligonucleotides having the base sequences shown in SEQ ID NOs: 10 and 11 as a primer set, to amplify the promoter of the kanamycin resistance gene. Subsequently, this PCR product was digested with *Bam*HI and *Kpn*I*,* and gel extraction was carried out to purify the promoter of the kanamycin resistance gene (Kmp). Further, a vector pUC 19 was digested with *Bam*HI and *Kpn*I*,* and gel extraction was carried out to purify pUC19. These *Bam*HI/KpnI-digested pUC19 and promoter of the kanamycin resistance gene were ligated to each other. The thus prepared plasmid was designated pKmp.

Subsequently, PCR was carried out using cells of the E. *coli* JM109 strain as a template and the oligonucleotides having the base sequences shown in SEQ ID NOs:12 and 13 as a primer set, to amplify the LDC gene. The amplified LDC gene was digested with *Nco*I and *Sac*I, and gel extraction was carried out to purify the LDC gene. Further, the plasmid pKmp was digested with *Nco*I and *Sac*I, and gel extraction was carried out to purify pKmp. These *Nco*I/*Sac*I-digested pKmp and LDC gene were ligated to each other. The thus prepared plasmid was designated pTM24.

Subsequently, PCR was carried out using cells of the ATCC13032 strain as a template and the oligonucleotides having the base sequences shown in SEQ ID NOs:14 and 15 as a primer set, to amplify the hom gene. The amplified hom gene was digested with *Sph*I and *Bam*HI, and gel extraction was carried out to purify the hom gene. Further, the above-described plasmid pTM38 was digested with *Sph*I and *Bam*HI, and gel extraction was carried out to purify pTM38. These *Sph*I/*Bam*HI-digested pTM38 and hom gene were ligated to each other. The thus prepared plasmid was designated pTM44.

Subsequently, PCR was carried out using the plasmid pTM24 as a template and the oligonucleotides having the base sequences shown in SEQ ID NOs:16 and 17 as a primer set, to amplify a Kmp-LDC gene fragment. The amplified Kmp-LDC gene fragment was digested with a restriction enzyme *Aor*51HI, and gel extraction was carried out to purify the Kmp-LDC gene fragment. pTM44 prepared as described above was digested with *Aor*51 HI, and gel extraction was carried out to purify pTM44. These *Aor*51HI-digested pTM44 and Kmp-LDC gene fragment were ligated to each other. The thus prepared plasmid was designated pTM45.

Subsequently, the plasmid pTM45 was introduced to the AK-1 strain by electroporation [FEMS Microbiology Letters, 65, p. 299 (1989)], and kanamycin-resistant strains were selected, followed by culturing the kanamycin-resistant strains on a medium supplemented with sucrose and selecting sucrose-resistant coryneform cells lacking the sacB gene by the double crossing-over method which is described in Biosci. Biotechnol. Biochem (2007), 71(9), 2130-5 as a method for preparing the TM45 strain. From the thus selected transformant, a genomic DNA solution was prepared according to a conventional method. Using this genomic DNA as a template and the oligonucleotides (SEQ ID NOs:18 and 19) as a primer set, PCR was carried out to obtain a product, which was then subjected to electrophoresis in 1.0% agarose gel. As a result, a single band of 3.5 kb was observed. By this, the selected transformant could be confirmed to have the LDC gene inserted at the hom locus. This transformant was designated the *Corynebacterium glutamicum* TM4552 strain (hereinafter referred to as the TM4552 strain for short).

(3) Preparation of Coryneform Bacterium Having Two Copies of LDC Gene (Example 1)

Thereafter, a plasmid for introduction of the LDC gene into the lactate dehydrogenase (LDH) locus was prepared.

(i) Preparation of LDC Gene Expression Cassette

As a promoter for expression of the LDC gene, the promoter (SEQ ID NO:2, hereinafter referred to as Pdiv for short) of the divIVA gene (SEQ ID NO:20) of the ATCC13032 strain was used.

First, PCR was carried out using as a template the genomic DNA of the ATCC13032 strain prepared according to a conventional method and the oligonucleotides having the base sequences shown in SEQ ID NOs:21 and 22 as a primer set, to clone Pdiv. For the PCR amplification reaction, KOD-plus polymerase (manufactured by Toyobo Co., Ltd.), and the reaction buffer, dNTP mix and the like attached to the polymerase were used. The reaction system was prepared such that 20 pmol/sample of the primers and 1 U/sample of KOD-Plus polymerase were contained in 50 $\mu$l of the reaction solution. Using a PCR amplification device iCycler (manufactured by BIO-RAD), the reaction solution was subjected to heat denaturation at 94°C for 5 minutes, followed by 30 cycles of 94°C (heat denaturation) for 30 seconds, 60°C (annealing of the primers) for 30 seconds and 68°C (extension of the complementary strand) for 30 seconds, after which the reaction solution was cooled to a temperature of 4°C. The primers for amplification of the gene (SEQ ID NOs:21 and 22) were prepared such that a *Bam*HI recognition site and an *Nco*I recognition site were attached to the 5'-end and the 3'-end, respectively.

The PCR amplification fragment was purified and the fragment was phosphorylated at its ends with T4 polynucleotide Kinase (manufactured by Takara Bio Inc.), followed by being ligated into pUC118 vector (which had been prepared by digestion with a restriction enzyme *Hinc*II and dephosphorylation of the cleavage site). The ligation was carried out using DNA Ligation Kit Ver.2 (manufactured by Takara Bio Inc.). The ligation solution was used to transform competent cells of E. *coli* DH5$\alpha$ (manufactured by Takara Bio Inc.), and the transformed cells were plated on an LB plate supplemented with 50 $\mu$g/mL ampicillin. The cells were cultured overnight. From grown colonies, plasmid DNA was recovered by miniprep, and the DNA was digested with restriction enzymes *Bam*HI and *Nco*I, followed by selecting plasmids to which the Pdiv fragment was inserted. All the series of operations were carried out according to the protocols attached to the products. The thus prepared plasmid was designated pCG5.

Subsequently, PCR was carried out using cells of the E. *coli* JM109 strain as a template, to clone the LDC gene. The PCR reaction was carried out in the same manner as described above, and the primers for amplification of the gene (SEQ ID NOs:23 and 24) were prepared such that an *Nco*I recognition site and a *Sac*I recognition site were attached to the 5'-end and the 3'-end, respectively. The obtained PCR amplification fragment comprising the LDC gene was purified and cloned into pUC118 in the same manner as described above. The prepared plasmid was designated pCG11.

Subsequently, pCG11 was digested with restriction enzymes *Nco*I and *Xba*I, and the fragment of about 2.1 kb containing the LDC gene was excised and purified, followed by ligating the fragment to pCG5 that had been preliminarily digested with restriction enzymes *Nco*I and *Xba*I. The obtained plasmid, which has a fragment in which the LDC gene is linked downstream of Pdiv, was designated pCG13.

(ii) Preparation of Plasmid to be Introduced into LDH Locus

As the homologous region to be introduced into the LDH locus, the 5'-end and 3'-end regions in the LDH gene each having a length of 500 bp were cloned. Each of the regions was subjected to PCR using the genome of the ATCC13032 strain as a template and SEQ ID NOs:25 and 26 (5'-end region) or SEQ ID NOs:27 and 28 (3'-end region) as a primer set in the same manner as described above. The primers for amplification of the 5'-end region (SEQ ID NOs: 25 and 26) were prepared such that a *Bgl*II recognition sequence was added to each of the 5'-end-side and the 3'-end-side, and the primers for amplification of the 3'-end region (SEQ ID NOs:27 and 28) were prepared such that an *Sal*I recognition sequence was loaded on the 5'-end-side and an *Sph*I recognition sequence was loaded on the 3'-end-side. The obtained PCR amplification fragments each having a length of about 500 bp and comprising the 5'-end or 3'-end region of the LDH gene were purified, and each of the purified fragments was cloned into pUC118 in the same manner as described above. The prepared plasmids were designated pCG28 (5'-end region) and pCG29 (3'-end region).

Subsequently, pCG13 was digested with restriction enzymes *Bam*HI and *Xba*I to excise the fragment of about 2.4 kb containing the Pdiv-LDC gene and the fragment was purified, followed by ligating the fragment to the above-described pTM38 that had been preliminarily digested with restriction enzymes *Bam*HI and *Xba*I. The obtained plasmid was designated pCG33.

Subsequently, pCG29 was digested with restriction enzymes *Sal*I and *Sph*I, and the fragment containing the 3'-end region of the LDH gene was excised and purified, followed by ligating the fragment to pCG33 that had been preliminarily digested with *Sal*I and *Sph*I. The obtained plasmid was designated pCG37.

Finally, pCG28 was digested with restriction enzymes *Bgl*II and *Bam*HI, and the fragment containing the 5'-end region of the LDH gene was excised and purified, followed by ligating the fragment to pCG37 that had been preliminarily digested with *Bgl*II and *Bam*HI. The obtained plasmid was designated pCG41.

(III) Introduction of pCG41 into Chromosome

To the above-prepared TM4552 strain, pCG41 prepared as described above was introduced, and transformants were selected on an agar medium containing LB (tryptone (10 g/l) (manufactured by Bacto), yeast extract (5 g/l) (manufactured by Bacto) and sodium chloride (10 g/l)) supplemented with kanamycin (25 μg/ml). Thereafter, the selected kanamycin-resistant coryneform cells were cultured on a medium supplemented with sucrose, and sucrose-resistant coryneform cells were selected by double crossing-over in the same manner as in Example 1. From the thus selected transformant, a genomic DNA solution was prepared. Using this genomic DNA as a template and the oligonucleotides (SEQ ID NOs:25 and 28) as a primer set, PCR was carried out, and the obtained product was subjected to electrophoresis in 1.0% agarose gel. As a result, a single band of about 3.3 kb was observed. It should be noted that a fragment of about 2 kb is expected in the case of the strain in which pCG41 is not introduced. Thus, it could be confirmed that the selected transformant has the Pdiv-LDC gene fragment inserted at the LDH locus. This transformant was designated the *Corynebacterium glutamicum* CG4541 strain (hereinafter referred to as the CG4541 strain for short).

(4) 1,5-Pentanediamine Fermentation Test

Using the TM4552 strain (Comparative Example 1) and CG4541 strain (Example 1) prepared as described above, a 1,5-pentanediamine fermentation test was carried out.

Into 5 mL of the medium shown in Table 1 which was sterilized, one platinum loop of each of the CG4541 strain (Example 3) and TM4552 strain (Comparative Example 1) was inoculated, and pre-preculture was carried out at 30°C for 24 hours with shaking. The entire volume of the obtained pre-preculture was inoculated into 45 ml of the same medium as in the pre-preculture, and preculture was carried out at 30°C at 120 rpm for 24 hours. Thereafter, the entire volume of the obtained preculture was inoculated into 1000 ml of the medium which is the same as the one shown in Table 1 except that the glucose concentration is 150 g/L, and culturing was carried out under aeration with sterilized air at 0.07 vvm at 30°C at a stirring blade rotation speed of 800 rpm at a controlled pH of 6.7. As neutralizers, an aqueous sulfuric acid solution (3 M) and aqueous ammonia (3 M) were used.

[Table 1]

| | | |
|---|---|---|
| Glucose | 50 | g/L |
| $(NH_4)_2SO_4$ | 20 | g/L |
| $NH_4Cl$ | 1 | g/L |
| $MgSO_4 \cdot 7H_2O$ | 0.4 | g/L |
| NaCl | 0.525 | g/L |
| $KH_2PO_4$ | 3 | g/L |
| $Na_2HPO_4$ | 6 | g/L |
| $CaCl_2 \cdot 2H_2O$ | 7.35 | mg/L |
| $FeSO_4 \cdot 7H_2O$ | 20 | mg/L |
| $MnSO_4 \cdot 4H_2O$ | 2 | mg/L, |
| $ZnSO_4 \cdot 7H_2O$ | 0.02 | mg/L |
| $CuSO_4 \cdot 5H_2O$ | 0.54 | mg/L |
| $(NH_4)_8Mo_7O_{24}.4H_2O$ | 0.08 | mg/L |
| $Na_2MoO_4 \cdot 2H_2O$ | 0.176 | mg/L |
| $Na_2B_4O_7 \cdot 7H_2O$ | 0.116 | mg/L |
| $FeCl_3.6H_2O$ | 1.74 | mg/L |
| $MnCl_2 \cdot 4H_2O$ | 0.0144 | mg/L |
| thiamine·HCl | 1 | mg/L |
| Biotine | 30 | μg/L |
| Protocatechuic acid | 15 | mg/L |
| homoserine | | mg/L |

The result of culturing of the CG4541 strain is shown in Fig. 1, and the result of culturing of the TM4552 strain is shown in Fig. 2. These results show that, in the case of the TM4552 strain (Comparative Example 1), the concentration of accumulated 1,5-pentanediamine stopped to increase in the middle of the culture, and accumulation of L-lysine as a precursor occurred. On the other hand, in the case of the CG4541 strain (Example 1), the concentration of accumulated 1,5-pentanediamine increased to not less than 10 g/L, and accumulation of L-lysine as a precursor did not occur at all.

(5) Measurement of Specific Activity of Lysine Decarboxylase

The LDH specific activities of the CG4541 strain (Example 1) and TM4552 strain (Comparative Example 1) cultured as described above were measured over time.

A 10-ml aliquot of the culture liquid of each strain cultured as described above was sampled over time, and the cells were collected by centrifugation at 4000 rpm for 5 minutes, followed by suspending the cells in 2 ml of a buffer (50 mM Tris-HCl, pH 8). In each of 2-mL screw-cap tubes (manufactured by WATSON), 0.4 g of glass beads (0.1 mm diameter, manufactured by As One Corporation) were placed, and 1 ml of the bacterial suspension was added thereto. Subsequently, homogenization at 4000 rpm for 1 minute was repeated 5 times, and the supernatant was collected by centrifugation at 12000 rpm for 5 minutes. The obtained supernatant was used as a raw enzyme liquid in the subsequent measurement of the specific activity of lysine decarboxylase. For measurement of the protein concentration, BCA Protein Assay Kit (manufactured by PIERCE) was used. The protein concentration in the raw enzyme liquid of the CG4541 strain is shown in Table 2, and the protein concentration in the raw enzyme liquid of the TM4552 strain is shown in Table 3.

[Table 2]

| Culture period | 0 hr | 23 hr | 45 hr | 65 hr | 96 hr | 118 hr | 141 hr | 159 hr |
|---|---|---|---|---|---|---|---|---|
| Protein concentration (g/L) | - | 3.2 | 2.4 | 1.9 | 2.2 | 3.1 | 3.2 | 3.0 |

[Table 3]

| Culture period | 0 hr | 25 hr | 45 hr | 69 hr | 90 hr | 112 hr | 135 hr | 159 hr |
|---|---|---|---|---|---|---|---|---|
| Protein concentration (g/L) | — | 9.3 | 11.2 | 11.0 | 10.2 | 9.4 | 8.4 | 6.0 |

Using the thus obtained raw enzyme liquid, the lysine decarboxylase activity was measured using L-lysine as a substrate. The composition of the reaction solution is shown in Table 4. The reaction was performed at 37°C for 30 minutes, and this was followed by boiling for 10 minutes for termination of the reaction. Using the supernatant obtained after centrifugation, the concentrations of L-lysine and 1,5-pentanediamine produced were measured by HPLC as described above, and the LDC specific activity per unit amount of protein was calculated according to Equation 1. Changes in the LDC specific activity over time are shown in Fig. 3.

[Table 4]

| Solution | Volume(μL) |
|---|---|
| Raw enzyme liquid | 395 |
| 500 mM L-lysine solution | 100 |
| 5 mM Pyridoxal-5-phosphate solution | 5 |
| Total | 500 |

As shown by the results shown in Fig. 3, the LDC specific activity of the TM4552 strain decreased with time during the course of the culturing, and could hardly be seen at Hour 90 of the culturing. On the other hand, although the LDC specific activity of the CG4541 strain showed a tendency to decrease with time during the culturing, a specific activity of not less than 180 mU/mg protein was maintained throughout the culturing.

Table 5 shows the LDC specific activity and the concentrations of 1,5-pentanediamine and L-lysine in the culture liquid observed for the CG4541 strain, and Table 6 shows the LDC specific activity and the concentrations of 1,5-pentanediamine and L-lysine in the culture liquid observed for the TM4552 strain.

[Table 5]

| Culture period | 0 hr | 23 hr | 45 hr | 65 hr | 96 hr | 118 hr | 141 hr | 159 hr |
|---|---|---|---|---|---|---|---|---|
| 1,5-PD in culture liquid (g/L) | 0 | 0.8 | 4.8 | 7.3 | 9.7 | 10.7 | 11 | 10.9 |
| L-lysine in culture liquid (g/L) | 0 | 0 | 0 | 0 | 0 | 0 | 0 | 0 |
| LDC specific activity (mU/mg protein) | — | 466 | 430 | 249 | 258 | 254 | 200 | 181 |

[Table 6]

| Culture period | 0 hr | 25 hr | 45 hr | 69 hr | 90 hr | 112 hr | 135 hr | 159 hr |
|---|---|---|---|---|---|---|---|---|
| 1,5-PD in culture liquid (g/L) | 0 | 0.7 | 3.7 | 5 | 5.3 | 5 | 1 4.9 | 5 |
| L-lysine in culture liquid (g/L) | 0 | 0 | 0.7 | 1.3 | 1.7 | 1.8 | 1.6 | 1.1 |
| LDC specific activity (mU/mg protein) | — | 83 | 39 | 11 | 4 | 5 | 3 | 3 |

As shown by the results shown in Table 6, in the case of the TM4552 strain, the LDC specific activity was 83 mU/mg protein and no accumulation of L-lysine in the culture liquid was observed at Hour 25 of the culturing, but the LDC specific activity decreased to 39 mU/mg protein and accumulation of L-lysine in the culture liquid was observed at

Hour 45 of the culturing. After this, the specific activity of LDC continued to decrease and accumulated L-lysine continued to increase. On the other hand, based on the results shown in table 5, in the case of the CG4541 strain, the LDC specific activity during the culturing was maintained at not less than 180 mU/mg protein, and no accumulation of L-lysine occurred at all in the culture liquid.

As promoters for expression of the LDC gene, the CG4541 strain uses the promoters of the kanamycin resistance gene and the divIVA gene. Since the LDC specific activity of the TM4552 strain, which uses only the promoter of the kanamycin resistance gene, was about 80 mU/mg protein even when the specific activity was highest, it is thought that the maintenance of high LDC specific activity in the CG4541 strain was due to the effect of the promoter of the divIVA gene.

INDUSTRIAL APPLICABILITY

The present invention is useful as a method for producing 1,5-pentanediamine, which can be used as a raw material for polyamides.

**Claims**

1. A method for producing 1,5-pentanediamine using a coryneform bacterium having a gene encoding lysine decarboxylase in its chromosome, which coryneform bacterium maintains lysine decarboxylase activity of not less than 50 mU/mg protein during culturing.

2. A method for producing 1,5-pentanediamine using a coryneform bacterium having a gene encoding lysine decarboxylase in its chromosome, said gene encoding lysine decarboxylase being linked downstream of a promoter that functions during the logarithmic growth phase.

3. The method according to claim 2, wherein said promoter is divIVA gene promoter.

4. The method according to claim 2 or 3, wherein said promoter is a promoter selected from (A) to (D) below:

   (A) a promoter having the base sequence shown in SEQ ID NO:2;
   (B) a promoter having the same base sequence as the base sequence shown in SEQ ID NO:2 except that one or several bases are substituted, deleted, inserted and/or added;
   (C) a promoter that entirely or partially hybridizes under stringent conditions with the promoter having the base sequence shown in SEQ ID NO:2 or the complementary strand thereof; and
   (D) a promoter having a base sequence having a sequence identity of not less than 80% to the base sequence shown in SEQ ID NO:2.

5. The method according to any of claims 1 to 4, wherein said gene encoding lysine decarboxylase is a gene derived from *E. coli.*

6. The method according to any of claims 1 to 5, wherein said gene encoding lysine decarboxylase is a gene selected from (A) to (D) below and encodes a protein having lysine decarboxylase activity:

   (A) a gene having the base sequence shown in SEQ ID NO:1;
   (B) a gene having the same base sequence as the base sequence shown in SEQ ID NO:1 except that one or several bases are substituted, deleted, inserted and/or added;
   (C) a gene that entirely or partially hybridizes under stringent conditions with a gene having the base sequence shown in SEQ ID NO:1 or the complementary strand thereof; and
   (D) a gene having a base sequence having a sequence identity of not less than 80% to the base sequence shown in SEQ ID NO:1.

7. The method according to any of claims 1 to 7, wherein said coryneform bacterium is a coryneform bacterium having an improved L-lysine productivity.

8. The method according to any of claims 1 to 8, wherein said coryneform bacterium has a mutant-type aspartate kinase having the same amino acid sequence as shown in SEQ ID NO:3 except that the 311 th amino acid residue is substituted by an amino acid other than threonine.

9. The method according to any of claims 1 to 8, wherein said coryneform bacterium has a decreased homoserine dehydrogenase activity or is deficient for homoserine dehydrogenase activity.

10. The method according to claim 9, wherein said coryneform bacterium is deficient for homoserine dehydrogenase activity because of insertional mutagenesis.

**11.** The method according to any of claims 1 to 10, wherein said coryneform bacterium is a bacterium belonging to the genus *Corynebacterium.*

**12.** The method according to claim 11, wherein said bacterium belonging to the genus *Corynebacterium* is *Corynebacterium glutamicum.*

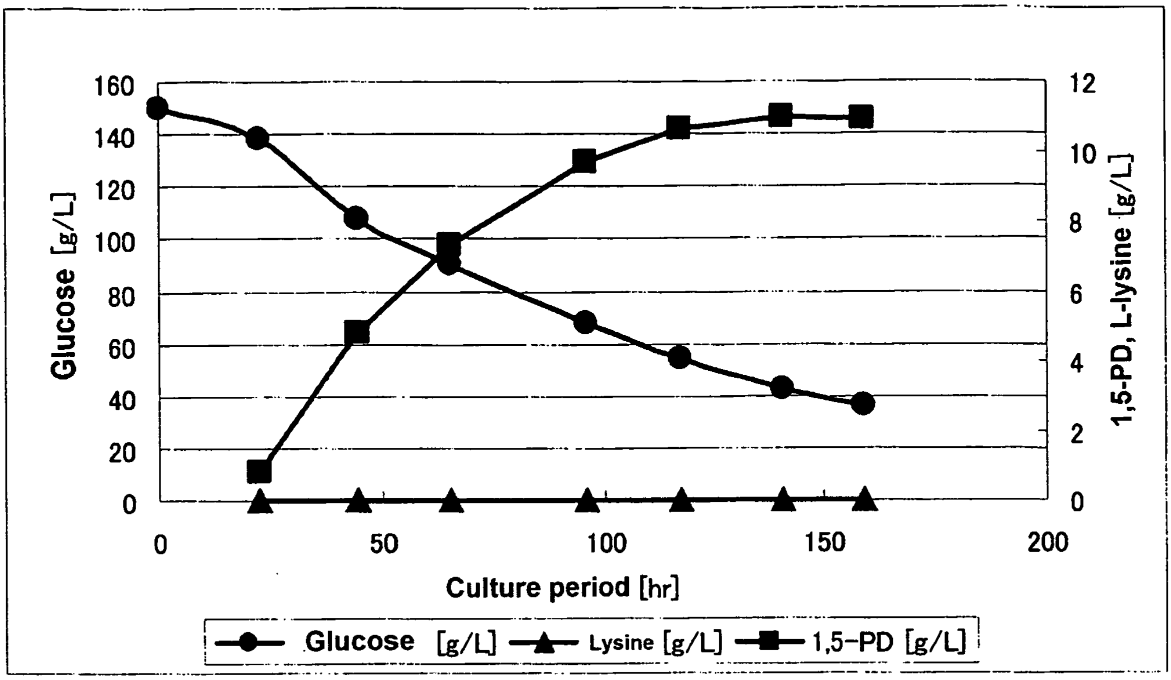
160
140
120
100
80
60
40
20
0
Glucose [g/L]
12
10
8
6
4
2
0
1,5-PD, L-lysine [g/L]
0
50
100
150
200
Culture period [hr]
Glucose [g/L]
Lysine [g/L]
1,5-PD [g/L]

Fig.1

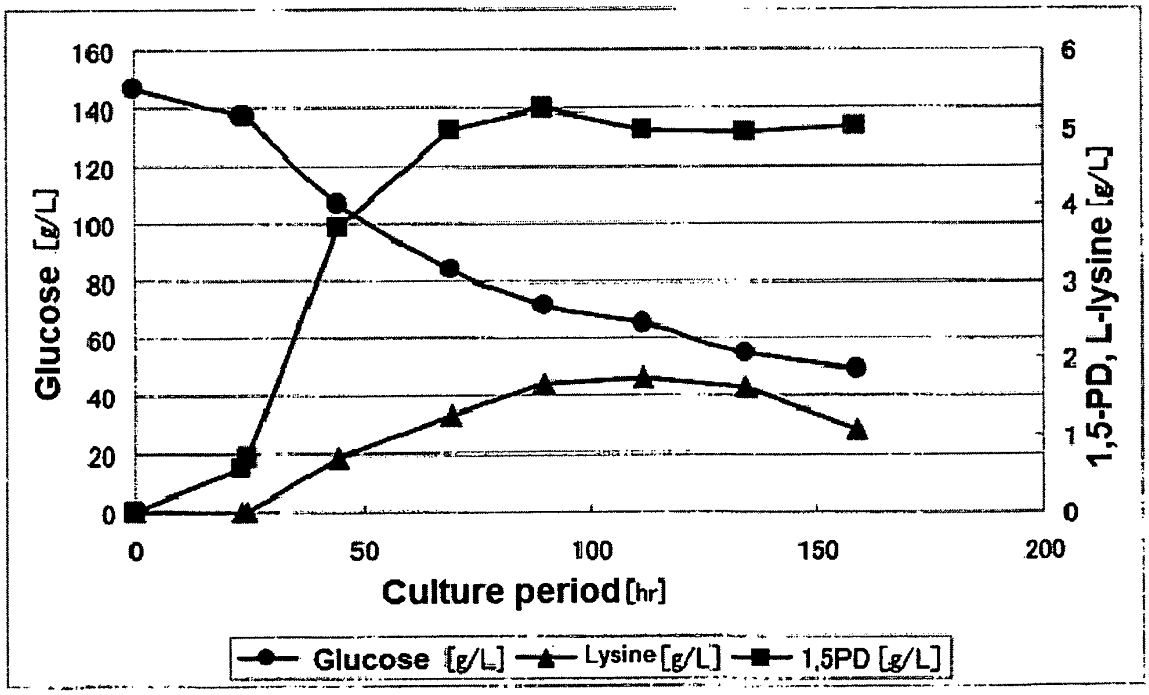
160
140
120
100
80
60
40
20
0
Glucose [g/L]
6
5
4
3
2
1
0
1,5-PD, L-lysine [g/L]
0
50
100
150
200
Culture period [hr]
Glucose [g/L]
Lysine [g/L]
1,5PD [g/L]

Fig.2

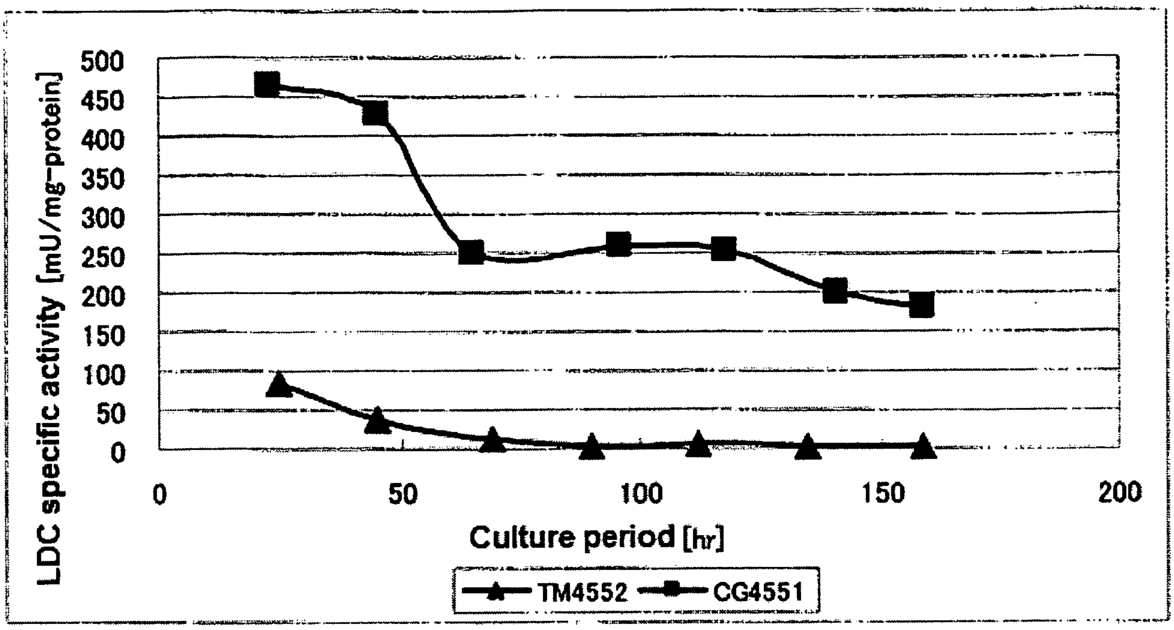
LDC specific activity [mU/mg-protein]
500
450
400
350
300
250
200
150
100
50
0
0
50
100
150
200
Culture period [hr]
TM4552
CG4551

Fig.3

**INTERNATIONAL SEARCH REPORT**

International application No.
PCT/JP2011/058987

A. CLASSIFICATION OF SUBJECT MATTER
*C12P13/00*(2006.01)i, *C12N15/09*(2006.01)i, *C07C211/09*(2006.01)n, *C12N9/88* (2006.01)n

According to International Patent Classification (IPC) or to both national classification and IPC

B. FIELDS SEARCHED

Minimum documentation searched (classification system followed by classification symbols)
C12P13/00, C12N15/09, C07C211/09, C12N9/88

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CA/REGISTRY(STN), JSTPlus/JMEDPlus/JST7580(JDreamII), PubMed, Science Direct, GenBank/EMBL/DDBJ/GeneSeq, SwissProt/PIR/GeneSeq, UniProt/GeneSeq

C. DOCUMENTS CONSIDERED TO BE RELEVANT

| Category* | Citation of document, with indication, where appropriate, of the relevant passages | Relevant to claim No. |
|---|---|---|
| X/Y | MIMITSUKA T et al., Metabolic Engineering of Corynebacterium glutamicum for Cadaverine Fermentation, Biosci Biotechnol Biochem., 2007, Vol.71(9), p2130-2135 | 1,2,5-7,9-12 /3,4,8 |
| Y/A | LETEK M, Role of DivIVA in the apical growth of Corynebacterium glutamicum, Universidad de Leon, 2007 | 3,4/1,2,5-12 |
| Y/A | OHNISHI J et al., A novel methodology employing Corynebacterium glutamicum genome information to generate a new L-lysine-producing mutant, Appl Microbiol Biotechnol., 2002, Vol.58, p217-223 | 8/1-7,9-12 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
31 May, 2011 (31.05.11)

Date of mailing of the international search report
07 June, 2011 (07.06.11)

Name and mailing address of the ISA/
Japanese Patent Office

Authorized officer

Facsimile No.

Telephone No.

Form PCT/ISA/210 (second sheet) (July 2009)

**INTERNATIONAL SEARCH REPORT**

International application No.
PCT/JP2011/058987

C (Continuation). DOCUMENTS CONSIDERED TO BE RELEVANT

| Category* | Citation of document, with indication, where appropriate, of the relevant passages | Relevant to claim No. |
|---|---|---|
| A | RAMOS A et al., Involvement of DivIVA in the morphology of the rod-shaped actinomycete Brevibacterium lactofermentum, Microbiology, 2003, Vol.149, p3531-3542 | 1-12 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002223770 A
- JP 2004222569 A
- JP 2009531042 PCT
- JP 2008104453 A
- JP 2009207495 A
- JP 2009029872 A
- JP 2009028045 A


### Non-patent literature cited in the description


- **SUYAMA ; KANEO.** *Yakugaku Zasshi,* 1965, vol. 85, 513-533
- **CELIA WHITE TABOR.** *Microbiological Reviews,* 1985, vol. 49, 81-99
- **TATENO et al.** *Appl Microbiol Biotechnol,* 2009, vol. 81 (1), 115-21
- **MIMITSUKA et al.** *Biosci Biotechnol Biochem,* 2007, vol. 71 (9), 2130-5
- Current Protocols I Molecular Biology. Publish. John Wily & Sons, 1987
- *Bio/Technology,* 1989, vol. 7, 1067-1070
- *Int. J. Syst., Bacteriol.,* 1981, vol. 41, 225
- *Journal of industrial Microbiol Biotechnol,* 2006, vol. 33 (7), 610-5
- *Appl. Microbiol. Biotechnol.,* 2002, vol. 58, 217-223
- *FEMS Microbiology Letters,* 1989, vol. 65, 299
- *Biosci. Biotechnol. Biochem,* 2007, vol. 71 (9), 2130-5